(19) Europäisches Patentamt
European Patent Office
Office européen des brevets

(11) Publication number: 0 354 434
A2

(12) **EUROPEAN PATENT APPLICATION**

(21) Application number: 89114086.5

(22) Date of filing: 31.07.89

(51) Int. Cl.4: C07C 255/50 , C07C 25/18 , C07C 43/205 , C07C 13/28 , C07C 49/792 , C07D 213/16 , C07D 213/803 , C07D 239/26 , C07C 253/30 , C07C 17/26 , C07C 41/30 , //C09K19/08

(30) Priority: 12.08.88 GB 8819224

(43) Date of publication of application: 14.02.90 Bulletin 90/07

(84) Designated Contracting States: CH DE FR GB IT LI

(71) Applicant: MERCK PATENT GESELLSCHAFT MIT BESCHRÄNKTER HAFTUNG
Frankfurter Strasse 250 Postfach 4119
D-6100 Darmstadt(DE)

(72) Inventor: Toyne, Kenneth Johnson, Dr.
25 Hall Road
Hull North Humberside HU6 8QW(GB)
Inventor: Hird, Michael, Dr.
222 Anlaby Park Road South
Hull North Humberside HU4 7BZ(GB)
Inventor: Greenfield, Simon, Dr.
2, Blackbird Close
Creekmoor Poole, BH17 7YA(GB)
Inventor: Poetsch, Eike, Dr.
Am Buchwald 4
D-6109 Mühltal(DE)

(54) **Process for the preparation of rod-like compounds.**

(57) Compounds of the formula I

$R^1-(A^o)_k-(Z^1-A^1)_m-Z^o-(E)_n-(A^2)_o-(Z^2-A^3)_p-R^2$ I

can be obtained from organoboron compounds of the formula II

$R^1-(A^o)_k-(Z^1-A^1)_m-Z^o-B(Y)_2$ II

and compounds of the formula III

$X-(E)_n-(A^2)_o-(Z^2-A^3)_p-R^2$ III

wherein is the compounds of the formulae I, II and III $A^o$, $A^1$, $A^2$, E, $R^1$, $R^2$, $Z^o$, Q, X, Y, K, m, n, o and p have the meaning given in claim 1 by coupling in the presence of a metal compound in high yields.

EP 0 354 434 A2

# Process for the preparation of rod-like compounds

The invention relates to a process for the preparation of compounds of the formula I

$R^1-(A^o)_k-(Z^1-A^1)_m-Z^o-(E)_n-(A^2)_o-(Z^2-A^3)_p-R^2$ I

wherein

$R^1$ and $R^2$ in each case independently of one another are CN, halogen or an alkyl residue or an alkenyl residue each with up to 18 C atoms optionally substituted by CN or at least one halogen atom, wherein one or two non-adjacent $CH_2$ groups of these residues can also be replaced by -O-, -CO-, -O-CO-and/or -CO-O-,

$A^o$, $A^1$, $A^2$ and $A^3$ are in each case independently of one another

a) a 1,4-cyclohexylene group, wherein one or two non-adjacent $CH_2$ groups can also be replaced by -O- and/or -S- and/or which can be substituted in the 1-position by $R^3$,

b) a 1,4-cyclohexenylene, a piperidine-1,4-diyl or 1,4-bicyclo-[2,2,2]-octylene group, or

c) a 1,4-phenylene group optionally substituted by $R^3$, wherein at least one CH group can also be replaced by N, E is $CR^3=CR^4$ or $CHR^3-CHR^4$,

$R^3$ and $R^4$ are in each case independently of one another H, alkyl with 1-6 C atoms, F, Cl, Br, $CF_3$ or CN,

$Z^o$ is a single bond or alkylene with 1-4 C atoms, wherein one or two $CH_2$ groups can be replaced by -CH(CN)-, -CH(F)- or -CH(Cl)- groups,

$Z^1$ and $Z^2$ are each -CO-O-, -O-CO-, $-CH_2CH_2-$, $-CHCN-CH_2-$, $-CH_2-CHCN-$, -CH=CH-, $-OCH_2-$, $-CH_2O-$, -CH=N-, -N=CH-, -NO=N-, -N=NO-or a single bond,

m and p are each 0, 1 or 2 and

n, o and k are each 0 or 1,

by means of organoboron compounds.

The processes known hitherto for the preparation of compounds of the formula I all lead to unsatisfactory results. Above all, poor yields and in some cases starting compounds which are accessible with difficulty are responsible for these. Thus, for example, the preparation of 1-cyano-2-fluoro-4-(trans-4-heptylcyclohexyl)-benzene, a compound of the formula I, according to EP 0,119,756 from the corresponding 1-acetyl compound takes place with a yield of only about 1 % of theory.

The preparation of 1-fluoro-2-(trans-4-alkylcyclohexyl) compounds by the method disclosed in U.S. 4,405,488 also requires considerable expenditure on synthesis and gives the desired products in only unsatisfactory yields.

Processes are furthermore known from DE 36 32 410 which enable organozinc compounds to be coupled with halogen compounds via transition metal catalysis. However, the coupling of organozinc compounds is accompanied by two major problems. First it is necessary to work at very low temperatures or with the use of ultrasound because the alkali metal derivatives which they are prepared from tend to suffer β-elimination at higher temperatures. Therefore, these procedures are to carry out only in safety reaction vessels or in very small amounts. The second problem is the fact that the resulting zinc compounds are very difficult to be separated from the reaction water. So these zinc compounds could reach the sewage and contaminate the environment.

Furthermore processes are known which enable phenylboronic acid to be coupled with haloarenes via palladium-catalyzed cross-coupling reaction in the presence of bases (for example, N. Miyaura, T. Yanagi, A. Suzuki, Synth. Comm. 11, 513-519 (1981). Also cross-coupling reactions between alkenylboranes and alkenyl-, alkynyl-, aryl-, allyl- and benzylhalides are known (for example A. Suzuki et al., Tetrahedron Lett., 3437 (1979); A. Suzuki et al., ibid, 2865 (1980)).

The object of the present invention was to discover a preparation process for the compounds of the formula I which has the disadvantages described for the previous processes to only a low degree, if at all.

Surprisingly, it has been found that organoboron compounds in the presence of a metal catalyst are outstandingly suitable for the preparation of compounds of the formula I.

The invention thus relates to a process for the preparation of compounds of the formula I, characterized in that an organoboron compound of the formula II

$R^1(-A^o)_k-(Z^1-A^1)_m-Z^o-B(Y)_2$ II

wherein $R^1$, $A^o$, $A^1$, $Z^o$, $Z^1$, k and m have the meaning given for formula I and Y is OH, alkyl or O-alkyl each with 1-6 C atoms, Cl, Br, I or a radical $R^1-(A^o)_k-(Z^1-A^1)_m-Z^o-$ with the above meaning or $(Y)_2$ denotes a group of the formulae a, b or c

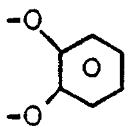 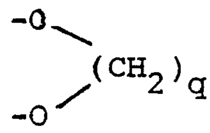 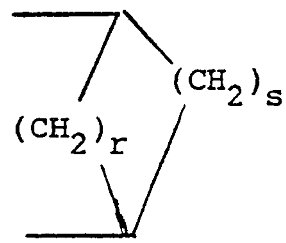

a b c

wherein q is 1, 2, 3 or 4, and r and s are each 2, 3, 4 or 5, is coupled in the presence of a metal compound with a compound of the formula III

$X-(E)_n-(A^2)_o-(Z^2-A^3)_p-R^2$ III

wherein $R^2$, E, $A^2$, $A^3$, $Z^2$, n, o and p have the meaning given for formula I and X is Cl, Br, I or $OSO_2-(CF_2)-q-CF_3$, wherein q denotes 0-10, with the provisos that

a) the sum of m, o, p and k is 2, 3, 4, 5 or 6,

b) $Z^2$ is $-CH_2-CH_2-$, $CHCN-CH_2-$, $-CH_2-CHCN$, -CH=CH-or a single bond if n + o = 0 and

c) $R^2$ is CN, in case at least one of $A^o$, $A^1$, $A^2$ and $A^3$ is a 1,4-phenylene group substituted by fluorine or CN.

The fact that such a stereoselective coupling of aliphatic organoboron compounds with aromatic, aliphatic or cycloaliphatic halogen compounds or sulfonates takes place was all the more surprising since in previously. known C-C coupling reactions $\beta$-elimination from the CH group adjacent to the coupling position occurs either during the preparation of the organometallic compound or from the organometallic compound itself as a side reaction which greatly influences the steric course and the yield.

The process according to the invention starts from the readily accessible metallizable compounds of the formula IV

$R^1-(A^o)_k-(Z^1-A^1)_m-Z^o-X'$ IV

wherein $R^1$, $A^o$, $A^1$, $Z^o$, k and m have the meaning given for formula I and X′ is chlorine, bromine, iodine, a hydrogen atom which can easily be separated off or a vinyl group which can easily be converted into a metal-ethylene group.

These are converted into the corresponding organoboron compounds of the formula II by known processes, such as are described, for example, in Houben-Weyl, Methoden der organischen Chemie (Methods of Organic Chemistry), Georg-Thieme-Verlag, Stuttgart, Volume 13/3a. The methods used are thereby preferably adapted to the nature of the functional groups in the metallizable compounds of the formula IV. Thus, for example, the preparation of organoboron compounds which are otherwise accessible only with difficulty is carried out, for example, by transmetallization of alkali metal derivatives in the manner described by Brown et al., Tetrahedron Lett. 1988, 2631 or by addition of an dialkylborane, for example 9-borabicyclo[3.3.1]nonane, to a compund of the formula IV wherein X′ denotes a vinyl group in the manner described by Suzuki et al., Tetrahedron Lett., 1986, 6369-6372.

The reaction of the metallizable compounds of the formula IV is preferably carried out in an inert solvent, for example ethers, such as diethyl ether, dimethoxyethane, tetrahydrofuran or dioxane, hydrocarbons, such as hexane, cyclohexane, benzene or toluene, or mixtures of these solvents with metallic lithium, magnesium turnings t-butyllithium, lithium naphthalenid or a strong organic base such as lithium diisopropylamide, potassium hexamethyldisilazone or lithium 2,2,6,6-tetramethyl piperidide. After completion of the metallization a boron compound is added at -50 °C to +50 °C preferably at 0 °C to +30 °C. Preferred boron compounds are boron trihalides, such as boron trichloride or boron tribromide or trialkylborates such as trimethylborate or triisopropylborate.

Under these conditions, the conversion of the metallizable compounds of the formula IV into the organoboron compounds of the formula II has usually ended within about 1 to 60 hours.

The coupling component of the formula III and a suitable metal catalyst is then added to the solution of the organoboron compound of the formula II, selective coupling to give the compounds of the formula I taking place.

The same solvents as mentioned above for the preparation of the organoboron compounds of the formula II are preferably used at the stated temperatures. The reaction times required are as a rule between about 1 and 50 hours.

Metallizable compounds of the formula IV and compounds of the formula III are used as the starting compounds. Halogen compounds which can be used are all the halides which can be converted into an

organoboron compound. The bromides, and furthermore also the chlorides or iodides, are preferably used.

The following formulae IVa to IVm represent a group of especially preferred starting compounds, it also being possible for the aromatic groups to be substituted by F, Cl, CN or $CH_3$ and for the cyclohexyl groups to be substituted in the 1-position by F, Cl, $CF_3$, CN or $C_1$-$C_4$ alkyl:

$R^1$-Br IVa

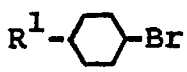 IVb

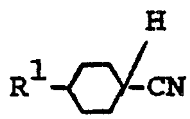 IVc

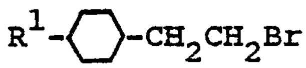 IVd

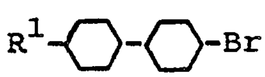 IVe

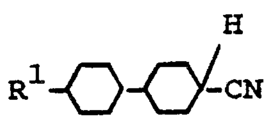 IVf

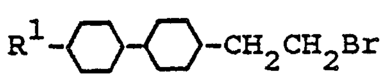 IVg

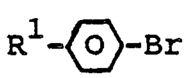 IVh

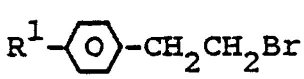 IVi

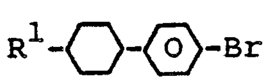 IVj

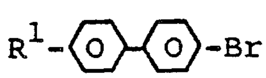 IVk

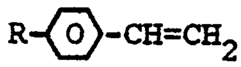

IVl

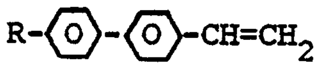

IVm

In these formulae, $R^1$ is preferably an alkyl group with 1-18 C atoms, wherein one of two non-adjacent $CH_2$ groups can also be replaced by -O- and/or -CO- and/or -CO-O-.

In the formulae I, II, III and IV, $R^1$ and $R^2$ are preferably straight-chain or branched alkyl with 1-18 C atoms, preferably with 2-10 C atoms, and accordingly are preferably ethyl, propyl, butyl, pentyl, hexyl, heptyl, octyl, nonyl or decyl, or furthermore also methyl, undecyl, dodecyl, tridecyl, tetradecyl or pentadecyl. Branched radicals, such as isopropyl, 2-butyl, isopentyl, 1-methylpentyl, 2-methylpentyl, 1-methylhexyl or 1-methylheptyl are also preferred. $R^1$ and $R^2$ are preferably also ethoxy, propoxy, butoxy, pentoxy, hexoxy, heptoxy, octoxy, nonoxy or decoxy, or furthermore also methoxy, undecoxy, dodecoxy, tridecoxy or pentadecoxy, or also isopropoxy, isopentoxy, 2-butoxy or 1-methylpentoxy.

All the compounds of the formula III can be used for coupling with the organoboron compounds of the formula II. The bromides (X = Br) are preferably used, and furthermore also the chlorides (X = Cl) and perfluoroalkane sulfonates ($X = OSO_2\text{-}(CF_2)_{0-10}\text{-}CH_3$). Amongst the perfluoroalkane sulfates, the trifluoromethane-, perfluoroethane- and perfluoropropane sulfates are particularly preferred.

The following formulae IIIa to IIIo represent a group of especially preferred starting compounds, it also being possible for the aromatic groups to be substituted by F, Cl, CN or $CH_3$ and for the cyclohexyl groups to be substituted in the 1-position by F, Cl, $CF_3$, CN or $C_1\text{-}C_4$ alkyl:

$Br\text{-}R^2$ IIIa

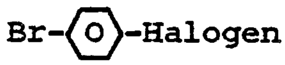

IIIb

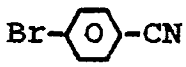

IIIc

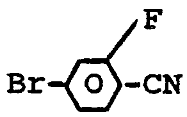

IIId

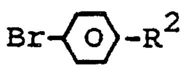

IIIe

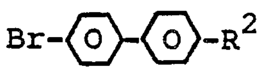

IIIf

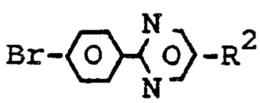

IIIg

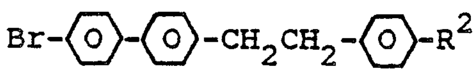

IIIh

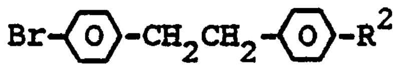

IIIi

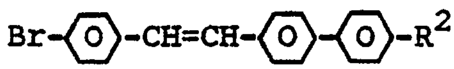

IIIj

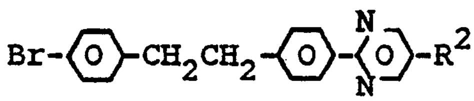

IIIk

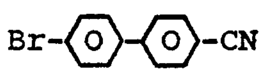

IIIl

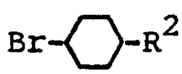

IIIm

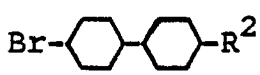

IIIn

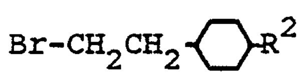

IIIo

In these formulae, $R^2$ is preferably an alkyl group with 1-15 C atoms, wherein one or two non-adjacent $CH_2$ groups can also be replaced by -O- and/or -CO- and/or -CO-O-.

Metallic catalysts which are preferably used are those of palladium and/or nickel. Ligands which are capable of forming suitable complexes are to be found, for example, in the group of phosphines, such as triphenylphosphine, tritolylphosphine, tris-(4-diemthylaminophenyl)-phosphine or 1,2-bis-(diphenylphosphino)-ethane or 1,1′-bis-(diphenylphosphino)-ferrocene, or of diketones, such as acetylacetone or octafluoroacetylacetone.

Salts of the above mentioned metals, such as, for example, $LiPdCl_3$ or corresponding nickel salts, can also be used. Mixed complexes, such as bis-(triphenylphosphine)-nickel dichloride are furthermore also suitable.

The use of tetrakis-(triphenylphosphine)-palladium or of [1,1′-bis-(diphenylphosphine)ferrocenyl]-palladium as a catalyst represents a particularly preferred embodiment of the present invention.

The halogen compounds of the formula IV used a starting compounds are known or can be prepared by known methods, such as are described, for example, in Houben-Weyl, Methoden der Organischen Chemie (Methods of Organic Chemistry), Volume 5/3 and 5/4. It is thus possible, for example, for alcohols of the formula IV in which X′ is a hydroxyl group to be converted into the corresponding halides with a phosphorus trihalide or with triphenylphospine/carbon tetrahalide.

The compounds of the formula III to be used as coupling components are likewise known or obtainable by known methods.

For example, halides of the formula III wherein X is Br or I, n is O and Q is an aromatic radical can be obtained by replacing the diazonium group in a corresponding diazonium salt by Br or I.

Perfluoroalkylsulfonates of the formula III wherein X is $OSO_2$-$(CF_2)q$-$CF_3$ can be prepared by esterification of corresponding alcohols of the formula III wherein X is a hydroxyl group with perfluoroalkane sulfonic acids or their reactive derivatives. The corresponding perfluoroalkane sulfonic acids are known.

Suitable reactive derivatives of the perfluoroalkane sulfonic acids mentioned are, in particular, the acid

halides, above all the chlorides and bromides, and furthermore the anhydrides, for example also mixed anhydrides, azides or esters, in particular alkyl esters with 1-4 C atoms in the alkyl group.

The present invention thus provides a very advantageous process for simple stereoselective preparation of compounds of the formula I in high yields.

The compounds of the formula I are suitable for use as liquid crystal materials, such as are disclosed, for example, in DE 32 17 597 and DE 26 36 684, or can be used as intermediate products for the preparation of other liquid crystal compounds.

The following examples are intended to illustrate the invention in more detail without limiting it. m.p. = melting point; C = crystalline; N = nematic; I = isotropic. Percentage data above and below are percentages by weight.

Customary working up below means: extractions with weakly acid water and toluene, drying of the organic phase, evaporation and purification by chromatography.

Example 1

Step (i)

Preparation of 3-Pentylbiphenyl-4′-boronic acid

A few millilitres of a solution of 4-bromo-4′-pentylbiphenyl (0.033 m) obtained from biphenyl by Friedel Crafts acylation with pentanoylchloride, Huang Minlon reduction with hydrazine hydrate, potassium hydroxide in digol followed by bromination with bromine in chloroform) in dry tetrahydrofuran (35 ml) are added to a suspension of magnesium turnings (0.036 m) and tetrahydrofuran (5 ml). The mixture is warmed to 75-80 °C to produce a gentle reflux. When the reaction started, the aryl bromide solution is added dropwise at a steady state rate so as to maintain a gentle reflux. When the reaction is complete, the mixture is cooled to 15 °C and added in a nitrogen atmosphere to trimethyl borate (0.0363 m) at 15 °C, a slight exothermic reaction occurred. The reaction is stirred at room temperature for 16 hrs. A 20 % hydrochloric acid solution (50 ml) is slowly added to the reaction mixture, and the mixture is then separated. The aqueous solution is extracted with diethyl ether (2 x 50 ml) and the combined organic layers are washed with water, dried over sodium sulphate and evaporated to give a solid.

Step (ii)

Preparation of 4-Cyano-4″-n-pentyl-p-terphenyl

The product from Step (i) 4-bromobenzonitrile (0.0275 m), tetrakis(triphenylphosphine) palladium (0.64 g), sodium carbonate (32 ml of a 2 m), toluene solution (64 ml) and methylated spirits (20 ml) are heated under reflux with vigorous stirring for 5 h. After cooling, the organic layer is separated, washed with water, dried over sodium sulphate and evaporated to dryness. The crude product is purified by passing through a short chromatography column (15 g silica) using a 1:1 mixture of petroleum spirit and dichloromethan, and crystallised from ethyl acetate with C 130 ° N 239 ° I.

Analogously are obtained

4-cyano-4″-n-propyl-p-terphenyl, C 182° N 257° I
4-cyano-4″-n-butyl-p-terphenyl, C 154° N 242° I
4-cyano-4″-n-hexyl-p-terphenyl, C 125° N 228° I
4-cyano-4″-n-heptyl-p-terphenyl, C 134° N 222° I

Example 2

Cis-4-(trans-4′-propylcyclohexyl)-cyclohexyl bromide (0.02 mol, prepared from the corresponding transalcohol with triphenylphosphine/bromine in acetonitrile in the manner known for bromination of alcohols) is treated with magnesium turnings (0.022 mol) in 50 ml of tetrahydrofuran. After the reaction being completed

the mixture is cooled to 15 °C and added to triisoprylborate (0.022 mol). After stirring the reaction mixture at room temperature for 20 hrs 0.16 g of tetrakis(triphenylphosphine)-palladium (0) and 4-bromo-2-fluorobenzonitrile (0.02 mol) are added to it and the mixture is stirred and heated under reflux for further 10 hours. It is then extracted with weakly acid water and toluene and the organic phase is dried over $MgSO_4$ and evaporated. Chromatography of the evaporation residue over silica gel with petroleum ether/diethyl ether 9:1 and crystallization from ethanol gives 4-cyano-3-fluoro-1-[trans-4′-(trans-4″-propylcyclohexyl)-cyclohexyl]benzene (54 % of theory) of melting point 54.3 °C and clear point 203 °C.

4-cyano-3-fluor-1-[trans-4′-(trans-4″-butylcyclohexyl)cyclohexyl]-benzene is obtained in an analogous manner from (0.02 mol) of cis-4-(trans-4′-butylcyclohexyl)-cyclohexyl bromide and (0.02 mol) of 4-trifluoromethanesulfonyl-2-fluorobenzonitrile; C/N 67 °, N/I 197.4 °.

The following compounds can be prepared analogusly (the transition temperatures quoted related to the crystalline/nematic (C/N) and nematic/isotropic (N/I) transition.

4-[Trans-4′-(trans-4″-alkylcyclohexyl)-cyclohexyl]-halogenobenzenes

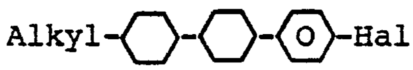

| Alkyl | Hal | C/N (°C) | N/I (°C) |
|---|---|---|---|
| $H_7C_3$ | F | 87.9 | 158.5 |
| $H_9C_4$ | F | 79.5 | 152.1 |
| $H_{13}C_6$ | Cl | 57.8 | 173.2 |

4-[Trans-4′-(trans-4″-alkylcyclohexyl)-cyclohexyl)-alkoxybenzenes and -alkylbenzenes

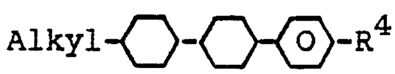

| Alkyl | $R^4$ | C/N (°C) | N/I (°C) |
|---|---|---|---|
| $H_7C_3$ | $OCH_3$ | 79.5 | 211 |
| $H_9C_4$ | $OC_6H_{13}$ | 70.0 | 186.3 |
| $H_7C_3$ | $CH_3$ | 64.6 | 179.8 |
| $H_{11}C_5$ | $C_3H_7$ | 48.6 | 181.0 |
| $H_7C_3$ | $C_5H_{11}$ | < 20 | 172.0 |

Trans-4-alkylcyclohexylaryl derivatives

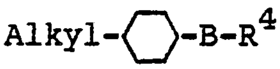

| Alkyl | B | $R^4$ | K/N (°C) | N/I (°C) |
|---|---|---|---|---|
| $H_7C_3$ | 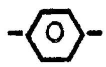 | 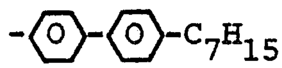 | | |
| $H_7C_3$ | 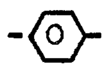 | 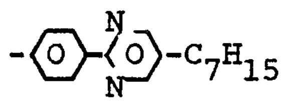 | 106 | 169 |
| $H_7C_3$ | 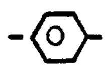 | 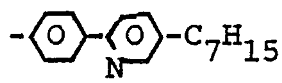 | | |
| $H_7C_3$ | 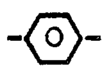 | 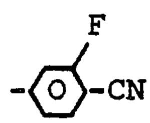 | | |
| $H_9C_4$ | 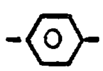 | 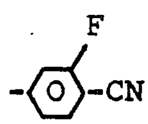 | | |
| $H_{11}C_5$ | 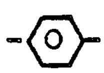 | 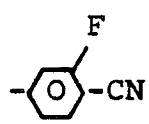 | | |
| $H_7C_3$ | 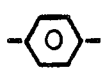 | 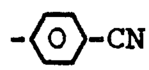 | | |
| $H_{15}C_7$ | 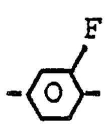 | -CN | 19 | 21 |

2-(4-(trans-4-alkylcyclohexyl)-phenyl)-5-alkylpyrimidines by coupling of 4-(trans-4-alkylcyclohexyl)-phenyl-boronic acid with 2-halo-5-alkylpyrimidines

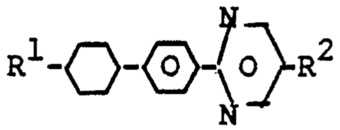

| $R^1$ | $R^2$ | K/N (°C) | N/I (°C) |
|---|---|---|---|
| $C_2H_5$ | $C_2H_5$ | 115 | 158 |
| $C_2H_5$ | $C_3H_7$ | 100 | 173 |
| $C_2H_5$ | $C_5H_{11}$ | 91 | 162 |
| $C_3H_7$ | $C_2H_5$ | 109 | 184 |
| $C_3H_7$ | $C_3H_7$ | 97 | 198 |
| $C_3H_7$ | $C_5H_{11}$ | 92 | 184 |
| $C_5H_{11}$ | $C_2H_5$ | 116 | 178 |
| $C_5H_{11}$ | $C_3H_7$ | 101 | 187 |
| $C_5H_{11}$ | $C_5H_{11}$ | 73 | 177 |

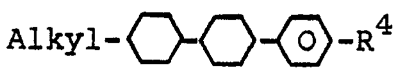

| Alkyl | $R^4$ | K/N (°C) | N/I (°C) |
|---|---|---|---|
| $C_3H_7$ | $COCH_3$ | 81 | 115 |
| $C_2H_5$ | CN | 76 | 195 |
| $C_3H_7$ | CN | 55 | 184 |
| $C_5H_{11}$ | 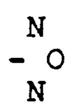 -$C_7H_{15}$ | 200 | 270 |

Example 3

The Grignard compound prepared from 1-bromo-2-(trans-4-pentylcyclohexyl)-ethane (0.02 mol) and magnesium (0.02 mol) in 40 ml of tetrahydrofuran is converted to 2-(trans-4-pentylcyclohexyl)-ethylboronic acid in an analogous manner as described in example 1, step (i). 4-Bromo-2-fluorobenzonitrile (0.02 mol) in 20 ml of tetrahydrofuran and 0.25 g of (0.02 mol) tetrakis(triphenylphosphine)-palladium(0) are then added.

After stirring overnight under reflux, the mixture is evaporated, the residue is extracted with water and diethyl ether, the organic phase is dried and evaporated and the residue is chromatographed over silica gel with petroleum ether/diethyl ether 9:1. After crystallization from methanol twice at -20 °C using active charcoal, the main fraction gives 1-(4-cyano-3-fluorophenyl)-2-(trans-4′-pentylcyclohexyl)-ethane. Melting point 16 °, clear point 21 °.

The following compounds can be prepared analogusly:

**1-Aryl-2-(trans-4'-alkylcyclohexyl)-ethanes**

Alkyl- Aryl

| Alkyl | Aryl | K/N (°C) | N/I (°C) |
|---|---|---|---|
| $H_7C_3$ | 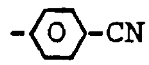 | 38 | 43 |
| $H_7C_3$ | 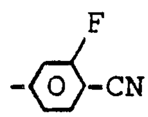 | | |

The corresponding 1-aryl-2-[trans-4′-alkylcyclohexyl)trans-4″-cyclohexyl]-ethanes are obtained by transmetallization of the corresponding Li compounds with triisopropylborate

| 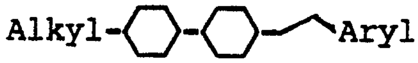 | | K/N (°C) | N/I (°C) |
|---|---|---|---|
| Alkyl | Aryl | | |
| $H_7C_3$ | 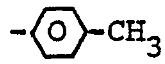 | 34 | 158 |
| $H_7C_3$ | 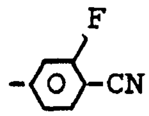 | 71.5 | 170 |
| $H_7C_3$ | 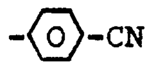 | 69 | 196 |
| $H_9C_4$ | 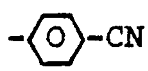 | K65 S75 N191 I | |
| $H_9C_4$ | 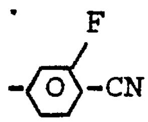 | 59 | 164.2 |
| $H_{11}C_5$ | 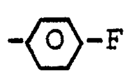 | K39 S107 N140 I | |
| $H_{11}C_5$ | 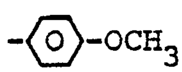 | K81 S158 N175.5 I | |

Example 4

Trans-4-(trans-4′-pentylcyclohexyl)-cyclohexane carbonitrile (0.02 mol) are dissolved in 7.5 ml of tetrahydrofuran and the solution is added dropwise, at -75 ° to -65 °C, to a solution of lithium diisopropylamide, which is prepared in 7.5 ml of tetrahydrofuran from 2.7 ml of diisopropylamine and 12.5 ml of a 15 % hexane solution of butyllithium. After the addition, the mixture is subsequently stirred for a further 30 minutes, and trimethyl borate (0.022 mol) dissolved in 10 ml of tetrahydrofuran, are then added dropwise, with further stirring. The mixture is subsequently stirred for 16 hrs at 50 °C, 50 mg of 1,2-bisdiphenylphosphinoethanonickel dichloride and 4-bromobenzonitrile (0.02 mol), dissolved in 15 ml of tetrahydrofuran, are then added, and after the cooling has been removed, the mixture is stirred under reflux for 5 hours. It is then decomposed with ice-water and evaporated, the residue is extracted with water/petroleum ether, the petroleum ether phase is dried and evaporated and the residue is chromatographed over silica gel with petroleum ether/diethyl ether 9:1. After recrystallisation twice from ethyl acetate, the main fraction gives trans-4-[trans-4′-(propylcyclohexyl)-cyclohexyl]-benzonitrile. The use of $NiCl_2$ -$(PPh_3)P_2$ under otherwise identical conditions gives comparable yields.

Melting point 113.3 °C, clear point: 135.6 °C, $\Delta\epsilon$ = 2.3

The following compounds are obtained analogously from the boron derivative of trans-4-(trans-4′-pentylcyclohexyl)-cyclohexanecarbonitrile:

| | melting point °C | clear point °C | Δε |
|---|---|---|---|
| 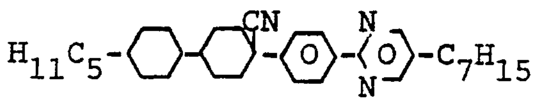 | 85.1 | 211.2 | -0.3 |
| 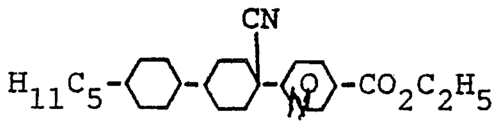 | 86.0 | 116.2 | -6.6 |

Example 5

Cis-4-(trans-4-pentyl)-cyclohexyl)-1-bromocyclohexane is converted to 4′-pentylbicyclohexyl-4-yl boronic acid in a manner described in Example 2. [1,1′-Bis-(diphenylphosphine) ferrocenyl]palladium(0), prepared from 0.57 g of [(diphenylphosphine)ferrocenyl]palladium-(II) chloride and 0.95 ml of a solution of diisobutylaluminium hydride in toluene (20 %) and trans-4-(trans-4-propylcyclohexyl)-1-bromocyclohexane (0.02 mol) in 50 ml of tetrahydrofuran are added and the mixture is stirred under reflux for 10 hours. Customary working up gives trans-4-(trans-4-(trans-4-propylcyclohexyl)cyclohexyl)-cyclohexyl)-1-pentylcyclohexane.

Example 6

4-(2-(trans-4-propylcyclohexyl)-ethyl)-phenylboronic acid (2.1 g, 0.0077 mol), 4-pentyl-2-fluorophenyl triflate (2.2 g, 0.007 mol) and tetrakis (triphenylphosphine), palladium (o) (0.16 g, 2 mol %) are heated in a mixture of triethylamine (5 ml) and dimethylformamide (20 ml) for 16 hours at 70° under nitrogen. The cooled reaction mixture is poured into water and extracted with ether (3 x 20 ml). The ether extracts are washed with water, dilute hydrochloric acid, water; aqueous $Na_2CO_3$ and water, then dried ($Na_2SO_4$) and the solvent evaporated to give 3.7 g of brown solid. The crude product is columned over silica/alumina (eluted with 40-60 °C petrol/dichloromethane) and then distilled to give 1.2 g unreacted and 0.8 g 2-(trans-4-propylcyclohexyl)-1-(4-pentyl-2-fluoro(biphenyl-4′-yl)ethane triflate (yield 30 %).

Analogously is obtained:

2-(trans,trans-4-propylbicyclohexyl-4′-yl)-1-(4-pentyl-2-fluorophenyl)-ethane.

**Claims**

1. Process for the preparation of compounds of the formula I

$R^1$-$(A^o)_k$-$(Z^1$-$A^1)_m$-$Z^o$-$(E)_n$-$(A^2)_o$-$(Z^2$-$A^3)_p$-$R^2$ I

wherein

$R^1$ and $R^2$ in each case independently of one another are CN, halogen or an alkyl residue or an alkenyl residue each with up to 18 C atoms optionally substituted by CN or at least one halogen atom, wherein one or two non-adjacent $CH_2$ groups of these residues can also be replaced by -O-, -CO-, -O-CO-and/or -CO-O-,

$A^o$, $A^1$, $A^2$ and $A^3$ are in each case independently of one another another

a) a 1,4-cyclohexylene group, wherein one or two non-adjacent $CH_2$ groups can also be replaced by -O- and/or -S- and/or which can be substituted in the 1-position by $R^3$,

b) a 1,4-cyclohexenylene, a piperidine-1,4-diyl or 1,4-bicyclo-[2,2,2]-octylene group, or

c) a 1,4-phenylene group optionally substituted by $R^3$, wherein at least one CH group can also be replaced by N,

E is $CR^3=CR^4$ or $CHR^3$-$CHR^4$,

$R^3$ and $R^4$ are in each case independently of one another H, alkyl with 1-6 C atoms, F, Cl, Br, $CF_3$ or

CN,

$Z^o$ is a single bond or alkylene with 1-4 C atoms, wherein one or two $CH_2$ groups can be replaced by -CH(CN)-, -CH(F)- or -CH(Cl)- groups,

$Z^1$ and $Z^2$ are each -CO-O-, -O-CO-, $-CH_2CH_2-$, $-CHCN-CH_2-$, $-CH_2-CHCN-$, -CH=CH-, $-OCH_2-$, $-CH_2O-$, -CH=N-, -N=CH-, -NO=N-, -N=NO-or a single bond,

m and p are each 0, 1 or 2 and

n, o and k are each 0 or 1,

characterized in that an organoboron compound of the formula II

$R^1-(A^o)_k(Z^1-A^1)_m-Z^o-B(Y)_2$ II

wherein $R^1$, $A^o$, $A^1$, $Z^o$, $Z^1$, k and m have the meaning given for formula I and Y is OH, alkyl or O-alkyl each with 1-6 C atoms, Cl, Br, I or a radical $R^1-(A^o)k-(Z^1-A^1)_m-Z^o-$ with the above meaning, or (Y) denotes a group of the formulae a, b, c

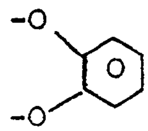 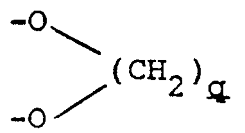 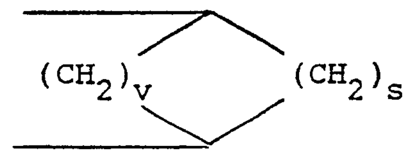

a b c

wherein q is 1, 2, 3 or 4, and v and s are each 2, 3, 4 or 5, is coupled in the presence of a metal compound with a compound of the formula III

$X-(E)_n-(A^2)_o-(Z^2-A^3))_p-R^2$ III

wherein $R^2$, E, $A^2$, $A^3$, $Z^2$, n, o and p have the meaning given for formula I and X is Cl, Br, I or $OSO_2-(CF_2)-q-CF_3$, wherein q denotes 0-10, with the provisos that

a) the sum of m, o, p and k is 2, 3, 4, 5 or 6,

b) $Z^2$ is $-CH_2-CH_2-$, $CHCN-CH_2-$, $-CH_2-CHCN$, -CH=CH-or a single bond if n + o = 0 and

c) $R^2$ is CN, in case at least one of $A^o$, $A^1$, $A^2$ and $A^3$ is a 1,4-phenylene group substituted by fluorine or CN.

2. Process according to Claim 1, characterized in that a compound of palladium and/or nickel is used as the metal compound.

3. Process according to Claim 1 or 2, characterized in that an organoboron compound of the formula II is coupled with a compound of the formula III′

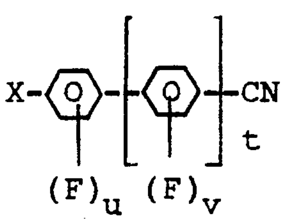

III'

wherein X has the meaning given, t is 0, 1 or 2, and u and v are each 0, 1 or 2.

4. Process according to claim 1 or 2 characterized in that an organoboron compound of the formula II1

$R^1-(A^o)_k-(Z^1-A^1)_m-Z^o-B(OH)_2$ II1

wherein $R^1$, $A^o$, $A^1$, $Z^1$, $Z^o$, K and m have the meaning given,

is coupled in the presence of a weak base with a compound of the formula III1

$F_3C-S(O)_2-O-(E)_n-(A^2)_o-(Z^2-A^3)_p-R^2$ III1

wherein $R^2$, $A^2$, $A^3$, E, $Z^2$, n, o and p have the meaning given.